# EUROPEAN PATENT APPLICATION

(11) **EP 1 413 284 A1**
(43) Date of publication of application: **28.04.2004**
(21) Application number: 02755659.6
(22) Date of filing: 25.07.2002
(51) Int. Cl.: A61K 7/02, A61K 7/00, A61K 7/027, A61K 7/032, A61K 7/043

(54) **COSMETIC**

(30) Priority: 01.08.2001 JP 2001233358
(71) Applicant: Nippon Sheet Glass Co., Ltd., Osaka-shi, Osaka 541-8559 (JP)
(72) Inventor: YOKOI, Koji,c/o Nippon Sheet Glass Co., Ltd., Osaka-shi, Osaka 541-8559 (JP); INO, Juichi,c/o Nippon Sheet Glass Co., Ltd., Osaka-shi, Osaka 541-8559 (JP); ISHIZUKA, Satoshi, c/o Nippon Sheet Glass Co., Ltd, Osaka-shi, Osaka 541-8559 (JP)
(74) Representative: Campbell, Neil Boyd
(86) International application number: PCT/JP2002/007553
(87) International publication number: WO 2003/011235

(57) **Abstract**

A cosmetic is provided that contains a powder that exhibits a shininess characteristic of a metal film and yet is not prone to leaching out of metal ions, whereby the cosmetic is not prone to causing discoloration or degeneration, and is not prone to bringing about a metal allergy. The powder contained in the cosmetic is comprised of a base material, and a metal film and a metal oxide film formed in this order on the base material.

## Description

### Technical Field

The present invention relates to a cosmetic for make-up containing a powder that has a metal film thereon and exhibits a glittery appearance.

### Background Art

Cosmetics for make-up are applied onto nails, around eyes, or onto lips, thus coloring or changing the impression of these body parts. In recent years, there has been a trend for a glittery impression to be desired, and hence the demand for cosmetics containing a lamé agent has increased. A powder in which a base material is coated with a metal, in particular a powder in which a base material consisting of minute plate-shaped particles such as mica or glass flakes is coated with a metal, exhibits a glittery high metallic luster, and when blended into a cosmetic, exhibits a marked effect as a lamé agent. Japanese Laid-open Patent Publication (Kokai) No. 62-108805 discloses a cosmetic having blended therein a precious-metal-containing pigment obtained by coating the surface of mica with gold, platinum, palladium, silver or the like using a chemical plating method. Japanese Laid-open Patent Publication (Kokai) No. 1-208324 discloses manufacturing a silver-material-coated inorganic powder by hydrolyzing a silver chloride to deposit silver oxide and/or silver hydroxide on the surface of an inorganic powder such as mica, talc, glass flakes, or aluminum flakes, and then carrying out heat treatment, and states that this powder is a pigment for cosmetics having an excellent make-up effect. Furthermore, Japanese Laid-open Patent Publication (Kokai) No. 2001-89324 discloses a cosmetic composition that contains glass particles having thereon a metal film of silver, nickel, chromium, molybdenum or the like.

However, according to these powders (pigments) having a metal film, the outermost surface is a metal, and hence there has been a problem that in the case of blending into a cosmetic, an organic component in the cosmetic may be discolored or degenerated through leaching out of metal ions or the manifestation of a catalytic function. Moreover, metal ions may bring about a metal allergy, and even though precious metals such as platinum and gold do not readily ionize, there have been reports that with uses in which there is direct contact with human skin such as with a cosmetic, a metal allergy may be brought about even though the metal leaches out only very slightly. Furthermore, in the case that the surface of a base material is plated with silver, the silver is attached in the form of minute particles, and hence undulations, albeit slight, are formed on the surface of the plating layer. There have been cases in which, when the powder is blended into a cosmetic, these slight surface undulations have an adverse effect on the feel or the spreadability on skin or nails.

### Disclosure of the Invention

The present invention has been accomplished focussing on problems such as the above. It is an object of the present invention to provide a cosmetic that contains a powder that exhibits a shininess characteristic of a metal film and yet is not prone to leaching out of metal ions, whereby the cosmetic is not prone to causing discoloration or degeneration, is not prone to bringing about a metal allergy, and is excellent in terms of feel and spreadability on skin or nails.

To attain the above object, according to an aspect of the present invention, there is provided a cosmetic containing a powder comprising a base material, and a metal film and a metal oxide film formed in this order on the base material.

In an aspect of the present invention, the metal film of the powder preferably has as a principal component thereof at least one metal selected from the group consisting of silver, gold, platinum, palladium, nickel, copper, chromium, molybdenum, tin, magnesium, aluminum, and hastelloy.

Moreover, in an aspect of the present invention, the base material of the powder preferably comprises glass flakes having a mean thickness in a range of 0.5 to 8.0µm and a mean particle diameter in a range of 5 to 1,000µm.

Moreover, in an aspect of the present invention, the metal oxide film of the powder preferably has as a principal component thereof at least one metal oxide selected from the group consisting of silica, alumina, zirconia, zinc oxide, and cerium oxide.

Moreover, in an aspect of the present invention, the powder preferably has the metal oxide film attached thereon in an amount in a range of 0.1 to 30wt% in terms of weight before formation of the metal oxide film.

### Best Modes for Carrying Out the Invention

A detailed description will now be given of embodiments of the present invention. However, the present invention is not limited to the following embodiments.

The present invention relates to a cosmetic having blended therein a powder in which a metal film formed on a base material is further covered with a metal oxide film, whereby the leaching out of metal ions is prevented and hence discoloration/degeneration and the occurrence of a metal allergy are prevented, while a highly glittery shininess is still exhibited.

A metal film exhibits a characteristic metallic luster, and hence a material comprised of a base material powder having a metal film provided thereon can be used as a shiny pigment. However, unless the problem of leaching out of metal ions described above is resolved, such a material is not desirable as a material of a cosmetic that is applied directly onto human skin. A metal oxide film is thus further provided on the metal film, whereby the leaching out of metal ions is prevented, without causing a deterioration in the metallic luster of the metal film.

The above function can be exhibited so long as the metal oxide film exists further to the outside than the metal film. It is thus necessary for the metal film and the metal oxide film to be formed in this order, but an intermediate film may be provided between the metal film and the metal oxide film, and/or a foundation film may be provided between the base material and the metal film, and/or a metal film and a metal oxide film may be built up on top of one another a plurality of times.

Moreover, if the metal oxide film covers a silver plating layer, then surface undulations on the plating layer are leveled out by the metal oxide film, and hence the surface smoothness of the powder increases. As a result, adhesion or agglomeration of the powder can be prevented, and hence the feel on skin or nails of a cosmetic containing the powder is improved, and the spreadability is improved.

So long as the base material is a powder, there are no particular limitations on the type or particle size thereof. For example, a commercially sold known powder can be used, for example mica, talc, kaolin, aluminum flakes, barium sulfate, silica flakes, alumina flakes, bismuth oxychloride, or the like. In particular, glass flakes having a mean thickness in a range of 0.5 to 8.0µm and a mean particle diameter in a range of 5 to 1,000µm are preferable. Glass flakes have desirable characteristics as a material of a cosmetic, for example the metallic luster is strong due to the surface smoothness being high, and adhesion and spreadability on skin are good due to being thin flakes. Furthermore, if the glass flakes have a thickness as stated above, then the strength required when blending or kneading a cosmetic can be secured, and hence the particle diameter will not become too small. Moreover, if the glass flakes have a particle diameter as stated above, then due to this and the surface smoothness, the glass flakes will be able to flow sufficiently in a cosmetic, and hence will be excellent in terms of attachment and adhesion to skin. In the case that texture is required where the cosmetic is applied, it is preferable for the particle diameter to be largish, whereas in the case that covering ability is required, it is preferable for the particle diameter to be smallish. In accordance with the objective, glass flakes having different particle diameters may be used mixed together. If glass flakes having a thickness and a particle diameter as stated above are used, then sufficient shininess can be obtained without applying a plurality of layers of the cosmetic on top of one another.

There are no particular limitations on the type of the metal film, but a metal film having as a principal component thereof at least one metal selected from the group consisting of silver, gold, platinum, palladium, nickel, copper, chromium, molybdenum, tin, magnesium, aluminum, and hastelloy is preferable. Of these, precious metals, i.e. silver, gold, platinum and palladium, are excellent in terms of shininess, and nickel is excellent in terms of shininess and color variation.

Examples of base material powders having such a metal film are, for example, the Metashine PS series having a film that has silver as a principal component thereof (Metashine RCFSX-5480PS, RCFSX-5230PS, RCFSX-5150PS, RCFSX-5090PS02, RCFSX-5030PS, REFSX-2040PS, REFSX-2025PS, REFSX-2015PS, etc. made by Nippon Sheet Glass Co., Ltd.), the Metashine GG series having a film that has gold as a principal component thereof (Metashine RCFSX-5150GG, etc. made by Nippon Sheet Glass Co., Ltd.), the Metashine NS series and NB series having a film that has nickel as a principal component thereof (Metashine RCFSX-5480NS, RCFSX-5230NS, RCFSX-5150NS, RCFSX-5090NS, etc., and RCFSX-5150NB, RCFSX-5090NB, etc. made by Nippon Sheet Glass Co., Ltd.), and the Crystal Star series having a film that has hastelloy as a principal component thereof (Crystal Star GF2525, etc. made by Toyo Aluminium K.K.).

The metal oxide film is preferably one having as a principal component thereof at least one metal oxide selected from the group consisting of silica, alumina, zirconia, zinc oxide, and cerium oxide. The metal oxide film may be a single film, or may be a film having a multi-layer structure. Moreover, in the case of mixing together raw materials as above and thus making the principal component be a compound material of two or more metal oxides, the combination of metal oxides can be selected as appropriate with an objective of improving the adhesion to the metal film or the compatibility with other materials in the cosmetic, or adjusting the refractive index. At a thickness of not more than several µm, any metal oxide film is transparent, and moreover the chemical stability is high, and hence the leaching out of metal ions can be suppressed, without marring the metallic luster of the metal film.

It is preferable to attach the metal oxide film in an amount in a range of 0.1 to 30wt% in terms of the weight of the powder before formation of the metal oxide film (this weight includes the base material and the metal film, and in some cases an intermediate film, and/or a foundation film, etc.). In the case that this attachment ratio is less than 0.1wt%, the function of preventing the leaching out of metal ions will be prone to becoming insufficient. On the other hand, if the attachment ratio exceeds 30wt%, then the metal oxide film will be prone to cracking and peeling off. Moreover, there will be an increase in the amount of raw materials used, and hence the cost will rise. Note also that, by suitably adjusting the thickness of the metal oxide film, the external appearance can be changed through interference of reflected light, and the metallic luster of the metal film can be given a subtle change of color.

There are no particular limitations on the method of forming the metal film and the method of forming the metal oxide film; a known method of forming a thin film on a powder can be used, for example, a method of depositing an oxide on the surface of the powder from a metal salt, a sol-gel method, a CVD method, or an LPD method. For example, the following are examples of methods of forming a silica film: as indicated in Japanese Examined Patent Application Publication (Kokoku) No. 46-9555, a method in which sodium silicate (water glass) is added to a slurry of the powder under an alkaline environment, thus depositing silica on the surface of the powder (metal salt method); as indicated in Japanese Examined Patent Application Publication (Kokoku) No. 48-32415 or Japanese Laid-open Patent Publication (Kokai) No. 3-54126, a method in which a mixture of the powder and a tetraalkoxysilane is put into a basic solution or an alkaline solution, thus forming a silica film on the surface of the powder through hydrolysis of the tetraalkoxysilane (sol-gel method); as indicated in Japanese Laid-open Patent Publication (Kokai) No. 3-066764, a method in which the powder is suspended in a silicofluoric acid solution, and then the equilibrium is shifted by adding boric acid or aluminum or raising the temperature, thus forming a silica film on the powder (LPD method). Similar methods can also be used for forming a film of another metal oxide such as alumina, zirconia, zinc oxide, or cerium oxide.

There are no particular limitations on the type of the cosmetic, provided that it is one that has contained a lamé agent, i.e. a shiny pigment, from hitherto. Examples are a nail cosmetic such as a nail color or a nail coating, an eye cosmetic such as an eye shadow, an eye liner, a mascara or an eyebrow pencil, a foundation, a rouge, a face color, a lipstick, a lip gloss, a pencil-shaped make-up cosmetic such as an eye liner pencil or a lip liner pencil, a precipitated-type make-up cosmetic in which a lamé agent is put in a precipitated state into water or a solvent and which is used after suitably shaking at the time of use, and so on.

The content of the powder in the cosmetic must be in a range of 0.1 to 100wt% (hereinafter abbreviated merely to "%"). The content of a pigment is generally determined by the formulation of the cosmetic, but the content is not limited to being as conventionally. Preferable contents of the powder in each of various formulations are as follows. For a nail cosmetic such as a nail color, 0.1 to 50% is preferable, and 3 to 40% is more preferable. At less than 0.1%, the function of the powder as a shiny pigment will be prone to becoming insufficient, whereas at more than 50%, the ability to apply the cosmetic may become poor. In the case of a solid powder cosmetic such as a rouge or an eye shadow that is obtained by carrying out dry filling of the powder by pressing or the like or by carrying out wet filling of the powder using a volatile solvent and then drying, 5 to 80% is preferable, and 10 to 60% is more preferable. If the powder content is within such a range, then the shininess of the powder is readily exhibited, and moreover the feeling in use is good. In the case of a powdery cosmetic such as an eye shadow or a face color that is used as a loose powder, the cosmetic mixes with oil present on a person's skin when used, and hence the powder content may be 100%. In the case of an oily solid cosmetic such as a lipstick or an oily eye shadow, 1 to 60% is preferable, and 3 to 50% is more preferable. At less than 1%, the shininess will be prone to becoming insufficient, whereas at more than 60%, the fluidity at high temperature will become poor, which is undesirable from the perspective of moldability. In the case of an emulsified make-up cosmetic obtained by emulsifying an aqueous phase and an oil phase together using a surfactant, 1 to 50% is preferable, and 3 to 40% is more preferable. At less than 1%, the shininess will be prone to becoming insufficient, whereas at more than 50%, the emulsification will be prone to becoming unstable. In the case of a water-based make-up cosmetic such as an aqueous mascara or an aqueous gel in which a watersoluble resin, an aqueous resin emulsion or a thickener or the like is blended into water, 0.1 to 60% is preferable, and 1 to 40% is more preferable. At less than 0.1%, the shininess will be prone to becoming insufficient, whereas more than 60% is undesirable from the perspective of usage.

The powder of the present invention may be subjected to hydrophobic modification as appropriate in accordance with the objective of the cosmetic. Examples of the method of carrying out the hydrophobic modification include treatment using a silicone compound such as methylhydrogenpolysiloxane, a high-viscosity silicone oil or a silicone resin, treatment using a surfactant such as an anionic surfactant or a cationic surfactant, treatment using a macromolecular compound such as nylon, polymethyl methacrylate, polyethylene, a fluororesin or a polyamino acid, treatment using a perfluoro-group-containing compound, lecithin, collagen, a metal soap, a lipophilic wax, or a polyhydric alcohol partial ester or complete ester, or a combination of these treatments. Note, however, that there is no limitation to the above methods; any method that can be used in the hydrophobic modification of a powder in general may be used.

Other components commonly used in cosmetics can be blended into the cosmetic as required. Examples of such other components are inorganic powders, organic powders, pigments, colorants, oily components, organic solvents, resins, plasticizers, and so on.

For example, examples of inorganic powders are talc, kaolin, sericite, muscovite, phlogopite, lepidolite, biotite, lithia mica, vermiculite, magnesium carbonate, calcium carbonate, diatomaceous earth, magnesium silicate, calcium silicate, aluminum silicate, barium silicate, barium sulfate, strontium silicate, metal tungstates, silica, hydroxyapatite, zeolite, boron nitride, ceramic powders, and so on.

Examples of organic powders are nylon powders, polyethylene powders, polystyrene powders, benzoguanamine powders, polytetrafluoroethylene powders, distyrene benzene polymer powders, epoxy powders, acrylic powders, and so on.

Examples of pigments are microcrystalline cellulose, inorganic white pigments such as titanium oxide and zinc oxide, inorganic red pigments such as red iron oxide and iron titanate, inorganic brown pigments such as γ-iron oxide, inorganic yellow pigments such as yellow iron oxide and loess, inorganic black pigments such as black iron oxide and carbon black, inorganic purple pigments such as mango violet and cobalt violet, inorganic green pigments such as chromium oxide, chromium hydroxide and cobalt titanate, inorganic blue pigments such as ultramarine blue and iron blue, pearl pigments such as titanium-oxide-coated mica, titanium-oxide-coated bismuth oxychloride, bismuth oxychloride, titanium-oxide-coated talc, fish scale flakes and colored titanium-oxide-coated mica, metal powder pigments such as aluminum powder and copper powder, and so on.

Examples of colorants are organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401 and Blue No. 404, organic pigments such as zirconium, barium and aluminum lakes such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3 and Blue No. 1, natural colorants such as chlorophyll and β-carotene, and so on.

Examples of oily components are squalane, liquid paraffin, vaseline, microcrystalline wax, OKEZORAITO, ceresin, myristic acid, palmitic acid, stearic acid, oleic acid, isostearic acid, cetyl alcohol, hexadecyl alcohol, oleyl alcohol, cetyl 2-ethylhexanoate, 2-ethylhexyl palmitate, 2-octyldodecyl myristate, neopentyl glycol di-2-ethylhexanoate, glyceryl tri-2-ethylhexanoate, 2-octyldodecyl oleate, isopropyl myristate, glyceryl triisostearate, cocoglycerides, olive oil, avocado oil, beeswax, myristyl myristate, various hydrocarbons such as mink oil and lanolin, silicone oils, higher fatty acids, esters of oils and fats, higher alcohols, waxes, and so on.

Examples of organic solvents are acetone, toluene, butyl acetate and other acetic acid esters, and so on; examples of resins are alkyd resins, urea resins, and so on; examples of plasticizers are camphor, acetyl tributyl citrate, and so on.

Furthermore, ultraviolet absorbers, antioxidants, preservatives, surfactants, moisturizers, fragrances, water, alcohols, thickeners and so on can also be selected as appropriate and blended in.

There are no particular limitations on the formulation of the cosmetic; examples are well-known forms such as a powder, a cake, a pencil, a stick, an ointment, a liquid, a milky lotion, or a cream.

The present invention will now be described in more detail through examples; however, so long as the scope of the present invention is not deviated from, there is no limitation to the following examples.

### Examples 1 to 6

First, a material selected as appropriate from the Metashine PS series of glass flakes coated with silver made by Nippon Sheet Glass Co., Ltd. was used as a base material powder having a metal film thereon, and a silica film was formed on the surface thereof using the following method.

50g of the Metashine PS was suspended in 0.51 of purified water, and while maintaining the temperature at 75°C using a water bath, a solution obtained by suitably weighing out a sodium silicate solution (silica solid content 37.1%, made by Wako Pure Chemical Industries, Ltd.) and then diluting by a factor of 10 with purified water was added slowly to the suspension. This was carried out while maintaining the pH of the suspension within a range of 9.2 to 9.4 using 3.5wt% hydrochloric acid. After the addition, stirring was continued for 30 minutes at 75°C. After that, the suspension was filtered using filter paper, the filtered off solid was recovered, and after washing with water and thus making the pH neutral, drying was carried out for 2 hours at 100°C, thus obtaining silver-coated glass flakes, i.e. a powder, having a silica film thereon. The silica film attachment ratio in each of the Examples was in a range of 1 to 20wt% relative to the Metashine PS, and specifically was as shown in Table 1 below.

The various powders prepared were evaluated using the following methods.

### Metal Leaching Tests

### Silver leaching test 1 (leaching out by water)

5g of each of the above powders was added to 95g of distilled water at 95°C so that the solid content was 5wt%, stirring was carried out for 30 minutes while maintaining the temperature at 95°C, and a 1-hour leaching test was carried out. After the test, filtration was carried out using a 0.23µm membrane filter, the amount of silver in the filtrate was determined by ICP optical emission spectroscopy, and the leaching rate per unit weight of the solid component was calculated. Silver leaching test 2 (leaching out by an organic solvent)

5g of each of the above powders was added to 95g of a 1:1 (weight ratio) mixed solvent of ethyl acetate and butyl acetate at 60°C so that the solid content was 5wt%, stirring was carried out for 30 minutes while maintaining the temperature at 60°C, and a 6-hour leaching test was carried out. After the test, the leaching rate was determined as described above. Organic matter discoloration test

A sample of butyl acetate, ethyl acetate, nitrocellulose and the powder mixed and stirred such that the butyl acetate : ethyl acetate : nitrocellulose : powder ratio was 50:25:20:5 (weight ratio) was put into a 10ml glass bottle and the bottle was sealed. The bottle was irradiated for 24 hours with a 200W mercury lamp, and the change in the color of the solution was visually observed. The results are shown in Table 1 below. Through this discoloration test, it can be recognized whether or not there will be discoloration when the powder is blended into a cosmetic containing organic matter.

**Table 1**

| Example | 1 | 2 | 3 | 4 | 5 | 6 |
|---|---|---|---|---|---|---|
| Mean thickness (µm) | 5.0 | 5.0 | 5.0 | 1.3 | 1.3 | 0.8 |
| Mean particle diameter (µm) | 480 | 90 | 30 | 80 | 15 | 20 |
| Silica film wt% | 1 | 3 | 3 | 3 | 6 | 15 |
| Silver leaching rate 1 (ppm) | 2.2 | 2.0 | 1.5 | 3.1 | 3.1 | 3.3 |
| Silver leaching rate 2 (ppm) | 1.2 | 1.2 | 0.8 | 0.7 | 0.6 | 0.8 |
| Discoloration of organic matter | None | None | None | None | None | None |

### Comparative Examples 1 to 3

Tests as in the Examples described above were carried out on various types of Metashine PS without forming a silica film. The results are shown in Table 2 below.

**Table 2**

| Comparative Example | 1 | 2 | 3 |
|---|---|---|---|
| Mean thickness (µm) | 5.0 | 1.3 | 1.3 |
| Mean particle diameter (µm) | 90 | 80 | 15 |
| Silica film wt% | 0 | 0 | 0 |
| Silver leaching rate 1 (ppm) | 9.2 | 131.2 | 261.1 |
| Silver leaching rate 2 (ppm) | 3.3 | 1.9 | 1.4 |
| Discoloration of Yellows slightly Yellows Yellows organic matter | | | |

From Table 1, it can be seen that for all of the powders having a silica film thereon, the leaching rate of silver into water is very low at not more than 3.3ppm, and the leaching rate of silver into the organic solvent is very low at not more than 1.2ppm. Moreover, it can be seen that because the leaching out of metal ions is suppressed, discoloration of organic matter does not occur.

On the other hand, from Table 2 it can be seen that for the glass flakes that do not have a silica film so that the silver film is exposed, the leaching rate of silver into water is very high at 9 to 261ppm, and the leaching rate of silver into the organic solvent is 1.4 to 3.3ppm, i.e. several times that for the case that a silica film is used. It can be seen that, as a result, in the case that such glass flakes not having a silica film are blended into a cosmetic, there is a high possibility that, due to leaching out of metal ions, a metal allergy will be brought about, and the cosmetic will discolor.

As described above, it can be seen that by providing a metal oxide film on the metal film, leaching out of metal ions can be suppressed, and accompanying this discoloration of organic matter can also be prevented.

### Example 7

50g of silver-coated glass flakes (Metashine Metashine PS made by Nippon Sheet Glass Co., Ltd.) having a mean thickness of 1.3µm and a mean particle diameter of 80µm were suspended in 0.51 of purified water, and while maintaining the temperature at 75°C using a water bath, a solution obtained by diluting 4g of a sodium silicate solution (silica solid content 37.1%, made by Wako Pure Chemical Industries, Ltd.) by a factor of 10 with purified water was added slowly to the suspension. This was carried out while maintaining the pH of the suspension within a range of 9.2 to 9.4 using 3.5wt% hydrochloric acid. After the addition, stirring was continued for 30 minutes at 75°C, thus forming a silica film on the silver film. The attachment ratio of the silica film was 3wt% relative to the silver-coated glass flakes, which corresponds to Example 4 above. Next, a solution obtained by diluting 1.8g of aluminum chloride hexahydrate by a factor of 20 with purified water was slowly instilled in. This was carried out while maintaining the pH within a range of 5.3 to 5.7 using a 5wt% sodium hydroxide aqueous solution. After the addition, stirring was continued for 30 minutes at 75°C, thus forming an alumina film (taking it as alumina, 2wt% relative to the silver-coated glass flakes). After that, the suspension was filtered using filter paper, the filtered off solid was recovered, and after washing with water and thus making the pH neutral, drying was carried out for 2 hours at 100°C. As a result of the above, pearlescent glass flakes, i.e. a powder, having a metal oxide film comprised of two layers, namely a silica film and an alumina film, were obtained.

For these silver-coated glass flakes having a silica-alumina two-layer coating film, the leaching rate of silver into water was 2.5ppm, and the leaching rate of silver into the organic solvent was 0.5ppm, and hence it can be seen that the leaching rates are lower than for Example 4. Moreover, discoloration of organic matter was not observed.

### Example 8

50g of nickel-coated glass flakes (Metashine NB made by Nippon Sheet Glass Co., Ltd.) having a mean thickness of 5.0µm and a mean particle diameter of 90µm were suspended in 0.51 of purified water, and while maintaining the temperature at 75°C using a water bath, a solution obtained by diluting 2.6g of zirconium oxychloride by a factor of 10 with purified water was added slowly to the suspension. This was carried out while maintaining the pH of the suspension at approximately 3 using a 1wt% sodium hydroxide solution. Stirring was continued for 30 minutes at 75°C, and then the pH of the suspension was raised to approximately 7.2 by adding the above sodium hydroxide solution, and stirring was continued for a further 30 minutes at 75°C. After that, the suspension was filtered using filter paper, the filtered off solid was recovered, and after washing with water and thus making the pH neutral, drying was carried out for 2 hours at 100°C, thus obtaining nickel-coated glass flakes, i.e. a powder, having a zirconia film (taking it as zirconia, 2wt% relative to the nickel-coated glass flakes).

For these nickel-coated glass flakes having a zirconia film, the leaching rate of silver into water was 1.8ppm, and the leaching rate of silver into the organic solvent was 0.3ppm.

In contrast, for such nickel-coated glass flakes not having a zirconia film, the leaching rate into water was 2.5ppm, and the leaching rate into the organic solvent was 0.7ppm. Moreover, in organic matter discoloration tests, for the nickel-coated glass flakes having a zirconia film, discoloration was not observed. It can thus be seen that a zirconia film is effective for suppressing the leaching out of nickel ions.

Next, a study was carried out into the feeling in use and so on for cosmetics having blended therein, as a lamé-type shiny pigment, metal-coated glass flakes having a metal oxide film thereon. The evaluation of the cosmetics was carried out by carrying out numerical conversion into points from 1 to 5 of the results of sensory evaluation at 5 levels by 10 panelists for the evaluation items shown in Table 3 below, and then taking the mean.

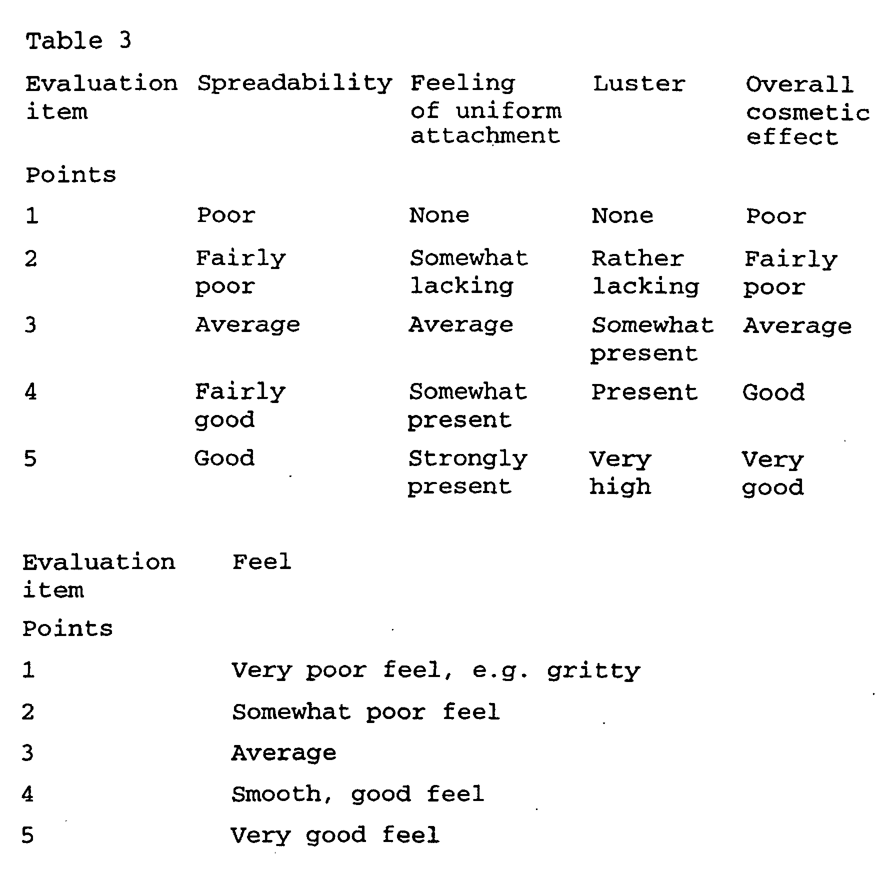

In the following Examples and Comparative Examples, to record the evaluation results in an easily understandable fashion, the mean values over the 10 panelists are represented using the following symbols.
ⓞ : At least 4.5, up to 5.0
○ : At least 3.5, but less than 4.5
● : At least 2.5, but less than 3.5
Δ : At least 1.5, but less than 2.5
× : At least 1.0, but less than 1.5

### Example 9: Nail enamel

A nail enamel of the following composition was manufactured using the following method.

| | |
|---|---|
| (1) Nitrocellulose | 12 |
| (2) Modified alkyd resin | 6 |
| (3) Acetyl tributyl citrate | 5 |
| (4) n-butyl acetate | 36.4 |
| (5) Ethyl acetate | 6 |
| (6) n-butyl alcohol | 2 |
| (7) Toluene | 20 |
| (8) Iron oxide pigment | 0.5 |
| (9) Titanium dioxide | 0.1 |
| (10) Glass flakes of Example 2 | 10 |
| (11) Mica | 1 |
| (12) Organic-modified montmorillonite | 1 (wt%) |

Components (1) to (7) (except only part of component (4)) were dissolved together, a gelated mixture of component (12) and the remainder of component (4) was added to the solution and mixing was carried out, components (8) to (11) were further added and mixing was carried out, and then the mixture was filled into a vessel, thus obtaining a nail enamel.

### Comparative Example 4: Nail enamel

A nail enamel was obtained as in Example 9, except that for component (10), the silver-coated glass flakes having a silica film were replaced with the silver-coated glass flakes not having a silica film of Comparative Example 1.

The results of the sensory tests for Example 9 and Comparative Example 4 are shown in Table 4 below.

**Table 4**

| Evaluation item | Feel Spreadability | Spreadability | Feeling of uniform attachment | Luster | Overall cosmetic effect |
|---|---|---|---|---|---|
| Example 9 | ⓞ | ○ | ⓞ | ⓞ | ⓞ |
| Comparative Example 4 | ○ | ○ | ⓞ | ⓞ | ⓞ |

From Table 4, it can be seen that the silica film on the silver-coated glass flakes is capable of improving the feel of a nail enamel, without having an adverse effect on the feeling in use of the nail enamel. Because the silica film suppresses the leaching out of silver ions as described earlier, according to the powder of the present invention, it is thus possible to suppress the occurrence of a metal allergy, and prevent discoloration of a nail enamel, without causing a deterioration in the feeling in use of the nail enamel.

### Example 10: Eye shadow

An eye shadow of the following composition was manufactured using the following method.

| | |
|---|---|
| (1) Talc | 12.6 |
| (2) Sericite | 8.1 |
| (3) Mica | 25.4 |
| (4) Glass flakes of Example 4 | 45.0 |
| (5) Red No. 226 | 0.4 |
| (6) Squalane | 3.0 |
| (7) 2-ethylhexyl palmitate | 5.0 |
| (8) Preservative | 0.3 |
| (9) Fragrance | 0.2 (wt%) |

Components (1) to (5) were mixed together using a Henschel mixer, a material obtained by mixing together components (6) to (9) with heating and melting was sprayed onto the mixture, further mixing was carried out, and then pulverization was carried out, and molding into a 4x6cm medium-sized dish was carried out, thus obtaining an eye shadow.

### Comparative Example 5: Eye shadow

An eye shadow was obtained as in Example 10, except that for component (4), the silver-coated glass flakes having a silica film were replaced with the silver-coated glass flakes of Comparative Example 2.

The results of the sensory tests for Example 10 and Comparative Example 5 are shown in Table 5 below.

**Table 5**

| Evaluation item | Feel Spreadability | Spreadability | Feeling of uniform attachment | Luster | Overall cosmetic effect |
|---|---|---|---|---|---|
| Example 10 | ⓞ | ⓞ | ⓞ | ⓞ | ⓞ |
| Comparative Example 5 | ○ | ○ | ⓞ | ⓞ | ⓞ |

From Table 5, it can be seen that the silica film on the silver-coated glass flakes is capable of improving the feel of an eye shadow, without having an adverse effect on the feeling in use of the eye shadow. Because the silica film suppresses the leaching out of silver ions as described earlier, according to the powder of the present invention, it is thus possible to suppress the occurrence of a metal allergy, and prevent discoloration of an eye shadow, without causing a deterioration in the feeling in use of the eye shadow.

### Example 11: Lipstick

A lipstick of the following composition was manufactured using a known method.

| | |
|---|---|
| (1) Paraffin | 12 |
| (2) Microcrystalline wax | 6 |
| (3) Hydrogenated polybutene | 20 |
| (4) Octyldodecanol | 10 |
| (5) caprylic/capric triglyceride | Suitable amount |
| (6) Red No. 202 | 0.1 |
| (7) Yellow No. 4 | 0.2 |
| (8) Barium sulfate | 1 |
| (9) Titanium oxide | 1 |
| (10) Glass flakes of Example 5 | 10 |

### Example 12: Lip gloss

A lip gloss of the following composition was manufactured using a known method.

| | |
|---|---|
| (1) Dextrin palmitate | 3 |
| (2) Heavy liquid isoparaffin | Suitable amount |
| (3) Isononyl isooctanoate | 10 |
| (4) Squalane | 5 |
| (5) Red No. 201 | 0.1 |
| (6) Ultramarine pink | 0.5 |
| (7) Barium sulfate | 0.1 |
| (8) Glass flakes of Example 6 | 10 |

### Example 13: Aqueous mascara

A lipstick of the following composition was manufactured using a known method.

| | |
|---|---|
| (1) Polyvinyl alcohol | 10 |
| (2) Carboxyvinyl polymer | 0.5 |
| (3) 1,3-butylene glycol | 5 |
| (4) Triethanolamine | 0.6 |
| (5) Ethanol | 5 |
| (6) Preservative | 0.2 |
| (7) Purified water | Suitable amount |
| (8) Glass flakes of Example 3 | 8 |

The results of the sensory tests for the cosmetics of Examples 11 to 13 are all shown together in Table 6 below.

**Table 6**

| Evaluation item | Feel Spreadability | Spreadability | Feeling of uniform attachment | Luster | Overall cosmetic effect |
|---|---|---|---|---|---|
| Example 11 | ⓞ | ⓞ | ⓞ | ⓞ | ⓞ |
| Example 12 | ⓞ | ⓞ | ⓞ | ⓞ | ⓞ |
| Example 13 | ⓞ | ○ | ⓞ | ⓞ | ⓞ |

From Table 6, it can be seen that in the case that silver-coated glass flakes having a silica film thereon are blended into a lipstick, a lip gloss or an aqueous mascara, there is no particular deterioration of the feeling in use. Furthermore, because the leaching out of silver ions is suppressed, a metal allergy is not brought about, and the cosmetic is not discolored.

### Industrial Applicability

As is clear from the above description, according to the cosmetic of the present invention, because the cosmetic contains a powder comprised of a base material, and a metal film and a metal oxide film formed in this order on the base material, leaching out of metal ions can be suppressed, and hence the occurrence of a metal allergy and the discoloration/degeneration of organic matter can be suppressed, while still exhibiting a metallic luster due to the metal film.

Moreover, by using glass flakes as the base material, a cosmetic according to which graininess is not conspicuous, shininess is very high, and the feeling in use, for example the feeling of uniform attachment and the spreadability on the skin, is excellent can be obtained using conventional means.

## Claims

1. A cosmetic containing a powder comprising a base material, and a metal film and a metal oxide film formed in this order on said base material.

2. A cosmetic as claimed in claim 1, wherein said metal film of said powder has as a principal component thereof at least one metal selected from the group consisting of silver, gold, platinum, palladium, nickel, copper, chromium, molybdenum, tin, magnesium, aluminum, and hastelloy.

3. A cosmetic as claimed in claim 1 or 2, wherein said base material of said powder comprises glass flakes having a mean thickness in a range of 0.5 to 8.0µm and a mean particle diameter in a range of 5 to 1,000µm.

4. A cosmetic as claimed in any one of claims 1 through 3, wherein said metal oxide film of said powder has as a principal component thereof at least one metal oxide selected from the group consisting of silica, alumina, zirconia, zinc oxide, and cerium oxide.

5. A cosmetic as claimed in any one of claims 1 through 4, wherein said powder has said metal oxide film attached thereon in an amount in a range of 0.1 to 30wt% in terms of weight before formation of said metal oxide film.
